# EUROPÄISCHE PATENTSCHRIFT

(11) **EP 2 582 415 B1**
(45) Veröffentlichungstag und Bekanntmachung des Hinweises auf die Patenterteilung: **14.05.2014**
(21) Anmeldenummer: 11738982.5
(22) Anmeldetag: 17.06.2011
(51) Int. Cl.: A61M 1/34

(54) **DURCHFLUSSFILTER ZUM SEPARIEREN VON BLUT IN PLASMA UND ZELLULÄRE BESTANDTEILE**
FLOW FILTER FOR SEPARATING BLOOD INTO PLASMA AND CELLULAR CONSTITUENTS
FILTRE TRAVERSANT SERVANT À SÉPARER LE SANG EN PLASMA ET COMPOSANTS CELLULAIRES

(30) Priorität: 17.06.2010 DE 102010030238
(43) Veröffentlichungstag der Anmeldung: 24.04.2013
(73) Patentinhaber: Lmb Lab med Blutbank Technologie GmbH, 85445 Schwaig (DE)
(72) Erfinder: STUTE, Reinhard, 66121 Saarbrücken (DE); JENTSCH, Klaus, 85445 Schwaig (DE)
(74) Vertreter: Winter, Brandl, Fürniss, Hübner, Röss, Kaiser, Polte - Partnerschaft
(86) Internationale Anmeldenummer: PCT/EP2011/060108
(87) Internationale Veröffentlichungsnummer: WO 2011/157822

(56) Entgegenhaltungen:
- EP-A1- 0 096 973
- EP-A1- 1 300 168
- EP-A1- 1 640 027
- WO-A1-83/00020
- WO-A2-2011/000834
- DE-U1- 20 014 311
- US-A1- 2005 184 012

## Beschreibung

Die vorliegende Erfindung betrifft einen Durchflussfilter zum Separieren von Blut in Plasma und zelluläre Bestandteile (Erythrozyten, Thrombozyten, Leukozyten) bzw. in eine Plasmafraktion und eine Fraktion zellulärer Bestandteile.

Filtervorrichtungen zum Separieren von Blut in bestimmte Fraktionen sind zum Beispiel aus den Druckschriften EP 1 309 363 B1, DE 102 39 658 A1, die EP 1 089 778 B1, DE 295 16 471 U1, EP 0 893 130 B1, EP 0 868 208 B1, DE 200 14 311 U1 und EP-A-0096973 bekannt.

In der in der EP 1 309 363 B1 beschriebenen Filtervorrichtung wird das einem Spender entnommene Blut durch ein in einem Filtergehäuse U-förmig angeordnetes Bündel aus Fasern, deren Wandungen aus einer feinporigen Membran gebildet sind, geleitet, wobei die Poren der Membran für die zellulären Bestandteile des Blutes zu klein sind, diese also durch die Fasern in ein entsprechendes Gefäß (z. B. einen Beutel) geleitet werden. Das Plasma hingegen tritt durch die Poren in das Filtergehäuse und wird von dort in ein weiteres Gefäß (z. B. einen Beutel) abgeführt. Diese Filtervorrichtung hat jedoch den Nachteil, dass die Veränderung des senkrecht zur Wandung wirkenden statischen Drucks, der letztlich für die Menge an Plasma, die durch die Poren hindurchtritt, und somit den Separationswirkungsgrad bestimmend ist, nicht berücksichtigt wird. Diese Veränderung ergibt sich aus der Zunahme der Dichte bzw. der Vergröβerung des Hämatokrits als Funktion des in den Fasern zurückgelegten Strömungsweges.

Es ist somit eine Aufgabe der vorliegenden Erfindung, den Separationswirkungsgrad zu verbessern und durch einfache konstruktive Maßnahmen einstellbar zu machen.

Diese Aufgabe wird durch die Merkmale der Ansprüche 1 bzw. 10 gelöst.

Gemäß der vorliegenden Erfindung umfasst ein Durchflussfilter zum Separieren von Blut in Plasma und zelluläre Bestandteile (1) ein Filtergehäuse, das eine Blutzuleitung, eine Plasma-Ableitung und eine Zell-Ableitung umfasst, (2) eine feinporige Membran, die (a) ein Bündel aus parallel geschalteten Hohlfasern umfasst, das in dem Filtergehäuse angeordnet ist und ein mit der Blutzuleitung verbundenes Einströmungsende aufweist, und (b) das Filtergehäuse in einen ersten Strömungsraum, der mit der Zell-Ableitung verbunden ist, und einen zweiten Strömungsraum, der mit der Plasma-Ableitung verbunden ist, unterteilt, und (3) eine Druckeinstellvorrichtung zur Einstellung des Drucks in den Hohlfasern, die mit einem Ausströmungsende des Bündels verbunden ist. Wie es unten detailliert beschrieben ist, lassen sich über eine Druckbeaufschlagung des Ausströmungsendes des Bündels die Druckverhältnisse in dem Bündel regulieren, indem dem in das Bündel bzw. das Filtergehäuse einströmenden Blut ein entsprechender Strömungswiderstand entgegengesetzt wird. Die Kurve des Druckverlaufs entlang jeder Hohlfaser des für die Separation des Plasmas entscheidenden statischen Drucks ist eine streng monoton steigende Funktion, die durch die Druckbeaufschlagung nach oben verschoben wird. Die Kurve der Strömungsgeschwindigkeit hingegen ist eine streng monoton fallende Funktion, die mit dem dynamischen Druck korrespondiert. Durch die Druckeinstellvorrichtung kann somit die "Ausbeute" an Plasma auf einfache Weise verändert werden, indem ein der Strömung des Bluts in dem Bündel entgegen wirkender Staudruck eingestellt wird. Eine Erhöhung des Staudrucks bewirkt ein langsameres Strömen und eine verbesserte Separation. Die Menge an Serum, die bei einem gegebenen Druck pro Zeiteinheit durch die Membran aus Hohlfasern dringt, kann natürlich stark durch die Porengröße beeinflusst werden.

Gemäß einer vorteilhaften Ausgestaltung der vorliegenden Erfindung umfasst die Druckeinstellvorrichtung eine Schlauchleitung, die mit einem externen Druck beaufschlagbar ist. "Extern" bedeutet, dass ein geeignetes Druckbeaufschlagungsmittel, mit dem das Bündel beaufschlagt wird, außerhalb des Durchlassfilters angeordnet ist und seine Wirkung über die Schlauchleitung ausübt. Vorteilhafterweise umfasst die Druckeinstellvorrichtung in diesem Fall ein Regelventil (Drosselventil), das in der Zell-Ableitung angeordnet und dazu geeignet ist, den Strömungsquerschnitt zu verändern. Alternativ (oder ergänzend) kann der Staudruck schlicht dadurch eingestellt werden, dass die Zell-Ableitung nach oben geführt und so der hydrostatische Druck des darin strömenden Plasma-armen Bluts ausgenutzt wird.

Gemäß der vorliegenden Erfindung umfasst die Druckeinstellvorrichtung eine zweite feinporige Membran, die ein zweites Bündel aus zweiten Hohlfasern umfasst, das in dem Filtergehäuse angeordnet ist und ein mit dem zweiten Ende des Bündels verbundenes erstes Ende und ein mit der Zell-Ableitung verbundenes zweites Ende aufweist. Die zweite feinporige Membran bzw. das zweite Bündel dient gemäß dieser Ausgestaltung als Druckeinstellvorrichtung; ihnen kommt somit eine Doppelfunktion zu: zweiter Filterschritt + Staudruckerzeugung. Die Orientierung des zweiten Bündels ist grundsätzlich von derjenigen des Bündels unabhängig. Vorteilhafterweise kann zur Erzeugung eines geeigneten Staudrucks dessen Strömungswiderstand, der durch seine vertikale Anordnung gebildete hydrostatische Druck und /oder ein Regelungsventil, wie es oben beschrieben ist, kombiniert werden. Vorteilhafterweise haben die Hohlfasern des Bündels eine andere Größe als die des zweiten Bündels, idealerweise sind die des Bündels kleiner als die des zweiten Bündels. Die kleinere Größe hat den Vorteil, dass nicht zu viel Plasma im ersten Strömungsabschnitt (dem Bündel) dem Blut entnommen wird, so dass dieses seine guten Strömungseigenschaften nicht verliert. Im zweiten Abschnitt (dem zweiten Bündel) hingegen, nachdem das Blut schon im Wesentlichen die Hälfte der Strecke zurückgelegt hat, sind die Poren größer, um das restliche Plasma besser aus dem Blut zu entnehmen.

Gemäß einer vorteilhaften Ausgestaltung der vorliegenden Erfindung sind die Anzahl der Hohlfasern und die Anzahl der zweiten Hohlfasern verschieden. Die Vorteile dieser Ausgestaltung sind unten im Zusammenhang mit einer konkreten Ausführungsform detailliert beschrieben.

Gemäß einer vorteilhaften Ausgestaltung der vorliegenden Erfindung sind das Bündel und das zweite Bündels jeweils mit ihrem ersten Ende und ihrem zweiten Ende so in dem Filtergehäuse befestigt, dass sich ihre Hohlfasern nicht berühren, und sind das Hohlfaserbündel und das zweite Hohlfaserbündel durch einen Verbindungsschlauch außerhalb des Filtergehäuses miteinander verbunden. Im Gegensatz zu dem in der EP 1 309 363 B1 beschriebenen Filter besitzt das erfindungsgemäße Durchlassfilter durch diese Konstruktion den Vorteil, dass das aus den Hohlfasern austretende Plasma nicht zu einem Aneinanderhaften (Adhäsion) der Hohlfasern führt, was in der EP 1 309 363 B1 leicht geschehen kann. Dadurch "kleben" die Hohlfasern aneinander, so dass die Poren im Endeffekt verstopft sind. Vorteilhafterweise wird der Abstand zwischen den Hohlfasern dadurch erreicht, dass deren erste und zweite Enden in gegenüberliegenden Wandabschnitten, zum Beispiel einer oberen Abdeckung und einer unteren Abdeckung, unter einer wohl definierten Vorspannung in dem Filtergehäuse befestigt sind.

Gemäß einer vorteilhaften Ausgestaltung der vorliegenden Erfindung umfasst das Filtergehäuse eine erste Kammer, in der das Bündel angeordnet ist, und eine gegenüber der ersten Kammer dichte zweite Kammer, in der das zweite Bündel angeordnet ist, wobei die Plasma-Ableitung mit der ersten Kammer und eine zweite Zell-Ableitung mit der zweiten Kammer verbunden ist. Vor dem Hintergrund der obigen Überlegungen hinsichtlich der Druckverhältnisse und Porengrößen ergibt sich hieraus der Vorteil, dass unterschiedliche Fraktionen separiert werden können. So ist es denkbar, dass die Leukozyten schon vor dem Filtergehäuse separiert werden und die Trennung von Plasma und verbliebener zellulärer Bestandteile wie Erythrozyten und Thrombozyten schon in der ersten Kammer zufriedenstellen erreicht wird. In der zweiten Kammer kann dann eine Trennung der letztgenannten zellulären Bestandteile aufgrund ihrer unterschiedlichen Größe erfolgen. Die in der zweiten Kammer separierten zellulären Bestandteile werden über die zweite Zell-Ableitung aus dem Filtergehäuse abgeführt.

Gemäß einer vorteilhaften Ausgestaltung der vorliegenden Erfindung erstrecken sich das Hohlfaserbündel und das zweite Hohlfaserbündel jeweils vertikal in dem Filtergehäuse. Dies hat den Vorteil, dass die Transportvorgänge allein durch die Gravitation bewirkt werden können.

Gemäß einer vorteilhaften Ausgestaltung der vorliegenden Erfindung ist in dem Verbindungsschlauch ein Sperrventil angeordnet. Das Sperrventil dient vorteilhafterweise der Verhinderung eines Rückflusses, wenn aus verfahrenstechnischen Gründen ein höherer Druck in dem zweiten Bündel erzeugt werden soll, der nicht auf das Bündel übertragen werden soll.

Gemäß einer vorteilhaften Ausgestaltung der vorliegenden Erfindung sind die Gröβe der Poren der ersten feinporigen Membran und die Größe der Poren der zweiten feinporigen Membran verschieden. Wie es oben schon beschrieben ist, ist die Porengröße neben der Anzahl an Hohlfasern des Bündels und der des zweiten Bündels etc. eine Möglichkeit, die Menge an Plasma und die Art der separierten Teile einzustellen.

Gemäß einer vorteilhaften Ausgestaltung der vorliegenden Erfindung ist die Blutleitung mit einem in einem vorbestimmten Abstand oberhalb des Filtergehäuses angeordneten Blutbeutel verbunden. Dies hat den Vorteil, dass der Eingangsdruck, das heißt der Druck beim Eintritt des Blutes in den Durchflussfilter - sofern keine weiteren Strömungshindernissen wie etwa ein Leukozytenfilter oder eine Tropfkammer in die Verbindung zwischen Durchflussfilter und Blutbeutel geschaltet sind - allein von dem Höhenunterschied abhängt, d. h. davon, wie weit der Blutbeutel über dem Durchflussfilter angeordnet ist. Diese Position kann vorteilhafterweise in Abhängigkeit von Eigenschaften der verwendeten Bündeln festgelegt werden.

Gemäß der vorliegenden Erfindung umfasst eine Filteranordnung (a) einen Blutbeutel, (b) einen Leukozytenfilter, (c) ein Durchflussfilter nach einem der Ansprüche 1 bis 9 und einen Zellbeutel und (d) einen Plasmabeutel wobei die Elemente (a) - (d) in dieser Reihenfolge auf zunehmend niedrigeren Niveaus angeordnet sind.

Gemäß einer vorteilhaften Ausgestaltung der vorliegenden Erfindung umfasst die Filteranordnung eine Byplassleitung zur Umgehung des Leukozytenfilters.

Die obigen und weitere Aufgaben, Eigenschaften und Vorteile der vorliegenden Erfindung sind aus der nachfolgenden detaillierten Beschreibung, die unter Bezugnahme auf die beigefügte Zeichnung gemacht wurde, deutlicher ersichtlich. In den Zeichnungen sind:
Fig. 1 eine beispielhafte Filteranordnung, in der das Durchflussfilter von Figur 2 gemäß der vorliegenden Erfindung verwendet werden kann; und
Fign. 3 bis 5 Ausführungsformen eines Durchlassfilters 100.

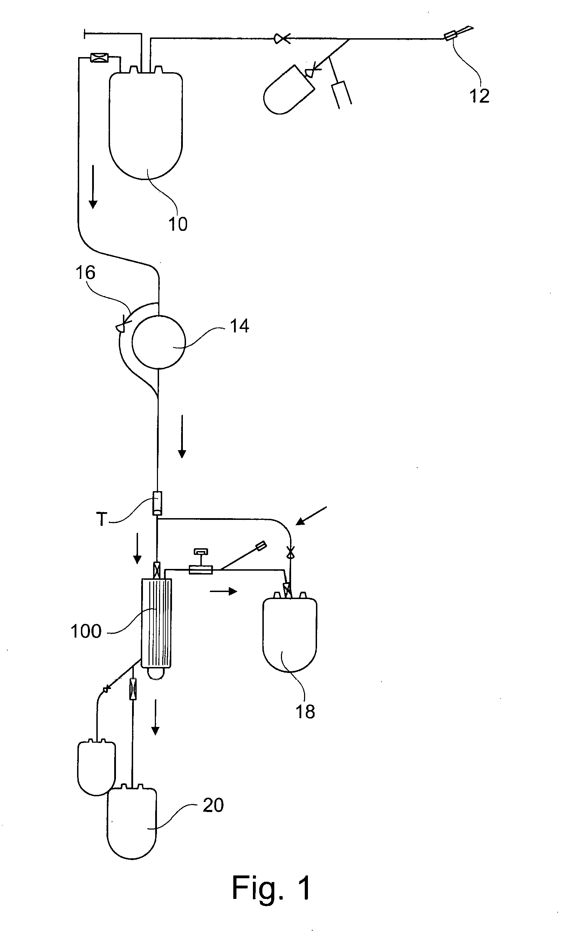

Fig. 1 zeigt eine beispielhafte Filteranordnung, in der das Durchflussfilter gemäß der vorliegenden Erfindung verwendet werden kann, wobei "oben" und "unten" in Fig. 1 ihre Entsprechung *realiter* in der vertikalen Anordnung der Komponenten finden, so dass das Vollblut (im Folgenden kurz als "Blut" bezeichnet) allein durch die Schwerkraft - entsprechend der eingezeichneten Pfeile - durch die Filteranordnung bewegt werden kann. Die Filteranordnung umfasst im Wesentlichen einen Blutbeutel 10, der mit einer Kanüle 12 verbunden ist, über die einem Spender (nicht gezeigt) Blut abgenommen wird. Unterhalb des Blutbeutels 10 und mit diesem über einen Schlauch verbunden ist ein Leukozytenfilter 14, der jedoch über eine Bypass-Leitung 16 umgangen werden kann. Unterhalb des Leukozytenfilters 14 und wiederum über einen Schlauch mit diesem verbunden befindet sich das erfindungsgemäße Durchlassfilter 100, das über Schläuche einerseits mit einem Erytrozytenbeutel 18, der neben dem Durchlassfilter 100 angeordnet ist, und andererseits mit einem Plasmabeutel 20, der unterhalb des Durchlassfilters 100 angeordnet ist, verbunden ist. Die Höhendifferenz zwischen dem Blutbeutel 10 und dem Durchlassfilter 100 beträgt etwa 100 cm, die zwischen dem Durchlassfilter 100 und dem Plasmabeutel etwa 50 cm. Durch diese Anordnung wird gravitationsbedingt am Eingang des Filtergehäuses 102 ein Druck von etwa 100 mbar erzeugt, der bis zum Ausgang des Filtergehäuses 102 auf etwa 10 mbar abfällt.

Über die Kanüle 12 ist der Spender mit der Filteranordnung verbunden. Das Blut läuft mit Hilfe der Kanüle 12 direkt in den Blutbeutel 10, in dem sich eine CPD (Citrat-Phosphat-Dextrose-) Stabilisatorlösung u. a. zur Verhinderung der Gerinnung des Bluts befindet.

Fig. 1 enthält weiterhin eine Tropfkammer T, verschiedene Ventile etc., die für die vorliegende Erfindung nur perifere Bedeutung haben und daher hier nicht eingehender beschrieben sind.

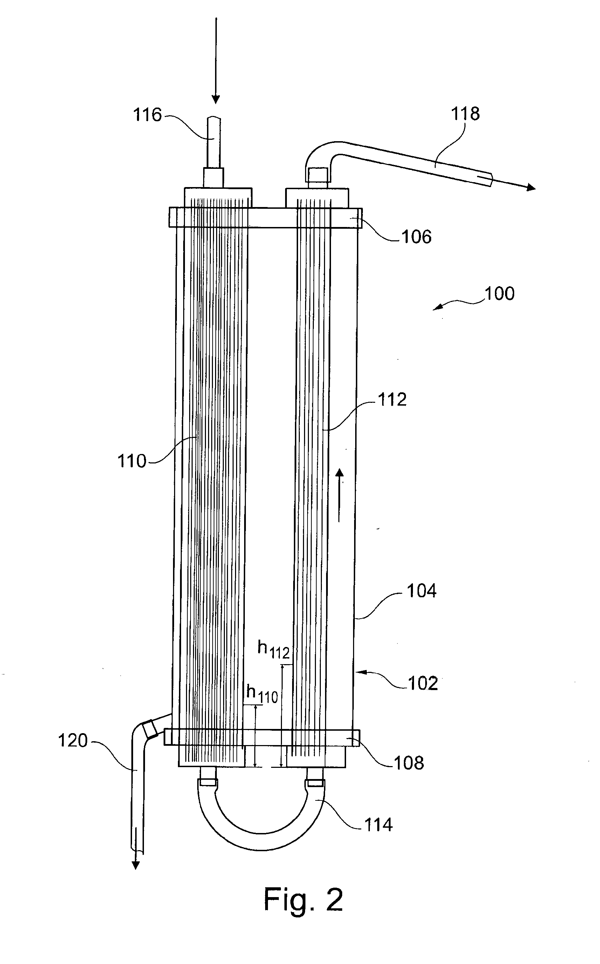

Fig. 2 zeigt eine vorteilhafte Ausführungsform des erfindungsgemäßen Durchlassfilters 100. Das Durchlassfilter 100 umfasst ein rohrförmiges Filtergehäuse 102 mit einer Wandung 104 in Form eines geraden Kreiszylinders, einer oberen Abdeckung 106 und einer unteren Abdeckung 108. In dem Filtergehäuse 102 und mit ihren jeweiligen oberen und unteren Enden in der oberen Abdeckung 106 bzw. der unteren Abdeckung 108 befestigt sind ein erstes Bündel 110 aus Hohlfasern und ein zweites Bündel 112 aus Hohlfasern parallel zueinander in einer vertikelen Ausrichtung angeordnet. Die Wandung jeder Hohlfaser ist aus einer feinporigen Membran gebildet. Wie es in Fig. 2 gezeigt ist, ist die Anzahl der Hohlfasern, die gemäß der Ausführungsform in beiden Bündeln 110, 112 identisch sind, in dem ersten Bündel 110 größer als in dem zweiten Bündel 112. Analoges gilt für deren Strömungsquerschnitte. Die Bündel 110, 112 sind unterhalb des Filtergehäuses 104 über einen Verbindungsschlauch 114 miteinander verbunden. Ferner ist das obere Ende des ersten Bündels 110 mit einer Blutzuleitung 116 zur Zuleitung des - gegebenenfalls von Leukozyten im Wesentlichen befreiten - Blutes und das obere Ende des zweiten Bündels 112 mit einer Zell-Ableitung 118 zur Ableitung der zellulären Bestandteile des Blutes verbunden. Darüber hinaus ist ein unterer Endabschnitt des Filtergehäuses 102 mit einer Plasma-Ableitung 120 zur Ableitung des Plasmas verbunden. Die Membranen der Hohlfasern trennen den Innenraum des Filtergehäuses 104 in einen ersten Strömungsraum, der mit der Zell-Ableitung 118 verbunden ist, und einen zweiten Strömungsraum, der mit der Plasma-Ableitung 120 verbunden ist. Die Gesamtlänge der Hohlfasern beider Bündel 110, 112 beträgt etwa 600 m, der Innendurchmesser jeder Faser etwa 300 µm, deren Wandstärke etwa 20 µm und deren Porendurchmesser etwa 0,7 µm. Im Vergleich dazu: Der Durchmesser eines Erytrozyten beträgt in etwa 7,5 µm, und die der Leukozyten liegt zwischen etwa 7,5 µm bei Lymphozyten und 20 µm bei Monozyten.

Das durch die Blutzuleitung 116 in das erste Bündel 110 einströmende Blut durchströmt unter kontinuierlicher Abgabe von Plasma durch die Poren der Membranwandungen der Hohlfasern und damit Erhöhung seiner Dichte zuerst das erste Bündel 110, dann den Verbindungsschlauch 114 und schließlich das zweite Bündel 112, um - idealerweise plasmafrei - über die Zell-Ableitung 118 in den Zellbeutel 18 zu gelangen. Entlang einer jeden Hohlfaser nimmt die Strömungsgeschwindigkeit durch das Austreten von Plasma aus der Hohlfaser kontinuierlich ab, während die Dichte des Bluts zunimmt, wie es oben bereits erwähnt ist. Da die Höhe h₁₁₀ eines aus dem unteren Endabschnitt des ersten Bündels 110 austretendes Differentialvolumens dV₁₁₀ kleiner als die entsprechende Höhe h₁₁₂ des entsprechenden in das zweite Bündel 112 eintretenden Differentialvolumens dV₁₁₂ ist, ist die Strömungsgeschwindigkeit am Ende des ersten Bündels 110 kleiner als die Strömungsgeschwindigkeit am Anfang des zweiten Bündels 112. Sie steigt am Übergang sprunghaft an, um dann wieder entlang jeder Faser des zweiten Bündels 112 kontinuierlich abzunehmen. Daraus ergibt sich, dass die pro Zeiteinheit gefilterte Plasmamenge am unteren Ende des ersten Bündels 110 und am oberen Ende des zweiten Bündels 112 am größten ist, da hier der statische Druck am höchsten ist. In dieser Anordnung erzeugt das zweite Bündel 112 durch den verengten Strömungsquerschnitt und die Beschleunigungsarbeit einen Staudruck im ersten Bündel 110. Die Höhe des Staudrucks wirkt bremsend zurück auf die Blutströmung in dem ersten Bündel 110, wodurch eine Regulierung des pro Zeiteinheit aus den Hohlfasern des ersten Bündels 110 austretenden Plasmas erreicht wird. Der Betrag dieses Staudrucks kann konstruktiv durch die Veränderung des Strömungsquerschnitts des zweiten Bündels 112 gegenüber dem ersten Bündel 110 bestimmt werden. Es ist zu beachten, dass auch durch den hydrostatischen Druck in dem zweiten Bündel 112 eine der Strömungsrichtung entgegengesetzte Kraft auf das Blut ausgeübt wird, sofern der Innendurchmesser der Hohlfasern so groß gewählt ist, dass der gravitative Effekt größer als der kohäsive ist. Das zweite Bündel 112 wirkt somit als die erfindungsgemäße Druckeinstellvorrichtung.

Es ist zu beachten, dass es vorteilhaft sein kann, die Bündel 110, 112 gegenüber der in Fig. 1 gezeigten Anordnung zu vertauschen, so dass im zweiten Bündel 112 mit der dann höheren Anzahl von Hohlfasern eine niedrigere Strömungsgeschwindigkeit und somit ein höherer statischer Druck vorliegt. Dadurch kann das Plasma, das am Ende des durch das Filtergehäuse 102 führenden Strömungsweges in nur noch geringer Menge vorhanden ist, effizienter entfernt werden.

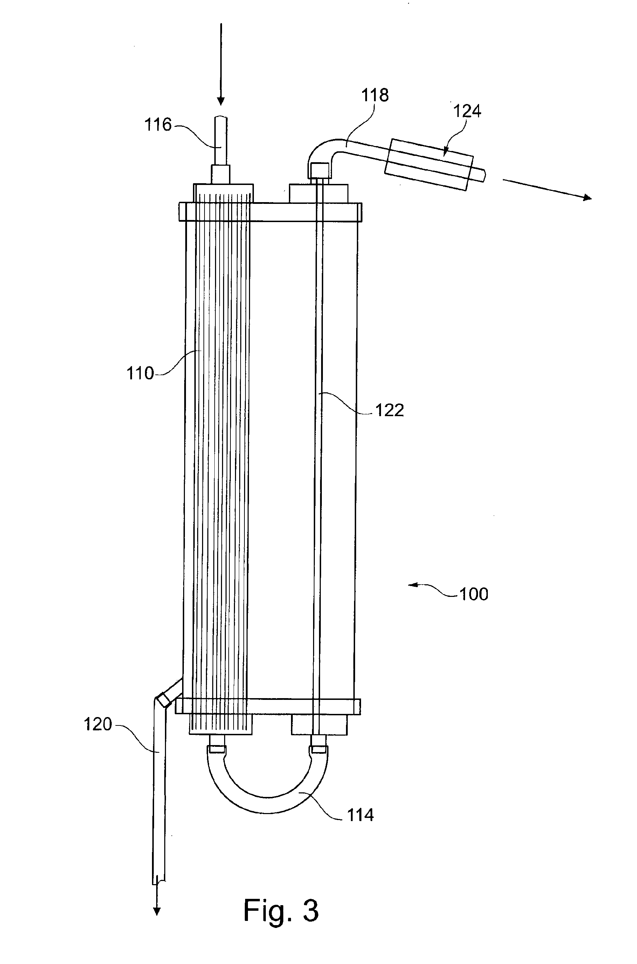

Fig. 3 zeigt eine weitere Ausführungsform eines Durchflussfilters 100. Der Durchflussfilter 100 gemäß dieser Ausführungsform unterscheidet sich von dem in Fig. 2 gezeigten dadurch, dass statt des zweiten Bündels 112 ein Steigrohr oder Steigschlauch 122 verwendet wird und zusätzlich ein Dosierventil 124 in der Zell-Ableitung 118 angeordnet ist. Das Steigrohr 122 und das Dosierventil 124 wirken gemäß dieser Ausführungsform zusammen als die erfindungsgemäße Druckeinstellvorrichtung, und zwar über den hydrostatischen Druck bzw. die Einstellung des Strömungsquerschnitts.

Den im Zusammenhang mit den Fign. 2 und 3 beschriebenen Ausführungsformen ist gemeinsam, dass dem hydrostatischen Druck, der durch den über dem erfindungsgemäßen Durchlassfilter 100 angeordneten Blutbeutel 10 erzeugt wird, ein einstellbarer Strömungswiderstand entgegengesetzt werden kann, welcher wiederum für die Druckverhältnisse in den Bündeln 110, 112 bestimmend ist.

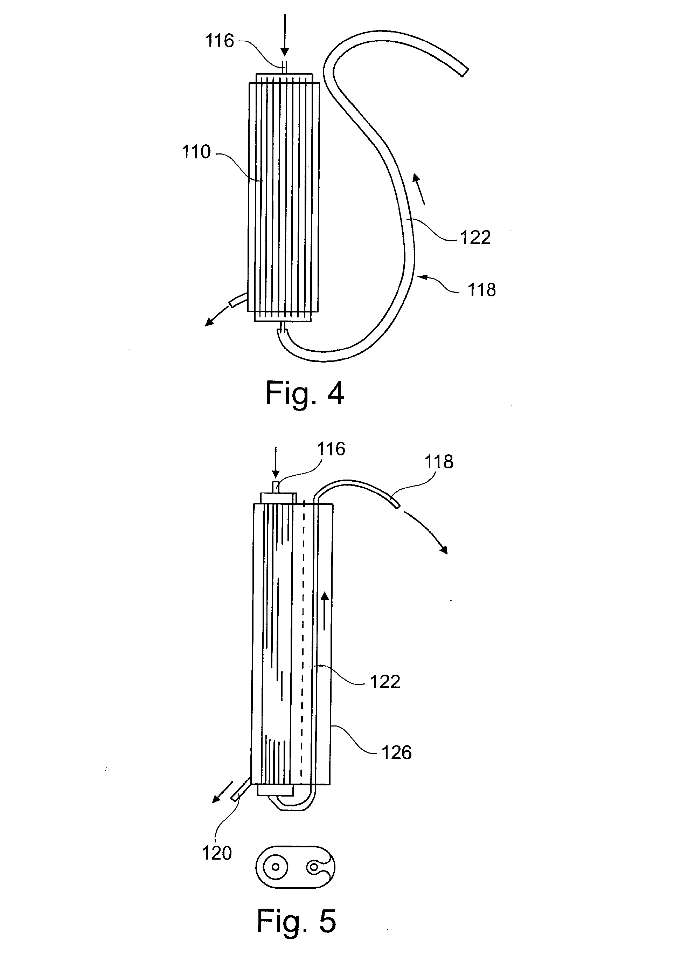

Fig. 4 zeigt eine weitere Ausführungsform, die sich von der in Fig. 3 gezeigten durch zwei Merkmale unterscheidet: (a) Das Steigrohr 122 ist außerhalb des Filtergehäuses 102 nach oben geführt, und (b) der dadurch in dem Gehäuse 102 frei gewordene Platz wird vollständig von dem ersten Bündel 110 eingenommen. Alternativ zu der in Fig. 4 gezeigten Variante, d. h. bei Vorhandensein von nur dem ersten Bündel 110 kann die Blutzuleitung auch entweder außerhalb oder innerhalb des Filtergehäuses 102 nach unten geführt und das Blut von unten in die Faser eingekoppelt werden, wodurch eine Strömungsumkehr erreicht wird.

Fig. 5 zeigt eine weitere Ausführungsform, die sich von der in Fig. 4 gezeigten dadurch unterscheidet, dass an dem Gehäuse 102 eine Führung 126 für das Steigrohr 122 befestigt - vorteilhafterweise einteilig mit diesem verbunden - ist, so dass das Steigrohr 122 nicht offenliegt, sondern geschützt aufgenommen ist.

### Bezugszeichenliste

- 10: Blutbeutel
- 12: Kanüle
- 14: Leukozytenfilter
- 16: Bypass-Leitung
- 18: Erytrozytenbeutel
- 20: Plasmabeutel
- 100: Durchlassfilter
- 102: Filtergehäuse
- 104: Wandung von 102
- 106: obere Abdeckung von 102
- 108: untere Abdeckung von 102
- 110: erstes Bündel
- 112: zweites Bündel
- 114: Verbindungsschlauch
- 116: Blutzuleitung
- 118: Zell-Ableitung
- 120: Plasma-Ableitung
- 122: Steigrohr
- 124: Dosierventil
- 126: Führung

## Patentansprüche

1. Durchflussfilter zum Separieren von Blut in Plasma und zelluläre Bestandteile, mit:
- einem Filtergehäuse, das eine Blutzuleitung, eine Plasma-Ableitung und eine Zell-Ableitung umfasst,
- einer feinporigen Membran, die
- ein Bündel aus parallel geschalteten Hohlfasern umfasst, das in dem Filtergehäuse angeordnet ist und ein mit der Blutzuleitung verbundenes Einströmungsende aufweist, und
- das Filtergehäuse in einen ersten Strömungsraum, der mit der Zell-Ableitung verbunden ist, und einen zweiten Strömungsraum, der mit der Plasma-Ableitung verbunden ist, unterteilt, und
- einer Druckeinstellvorrichtung zur Einstellung des Drucks in den Hohlfasern, die eine zweite feinporige Membran umfasst, die ein zweites Bündel aus zweiten Hohlfasern umfasst, das in dem Filtergehäuse angeordnet ist und ein mit einem Ausströmungsende des Bündels verbundenes erstes Ende und ein mit der Zell-Ableitung verbundenes zweites Ende aufweist.

2. Durchflussfilter nach Anspruch 1, **dadurch gekennzeichnet, dass** die Druckeinstellvorrichtung eine Schlauchleitung umfasst, die mit einem externen Druck beaufschlagbar ist.

3. Durchflussfilter nach Anspruch 1 oder 2, **dadurch gekennzeichnet**, die Anzahl der Hohlfasern und die Anzahl der zweiten Hohlfasern verschieden sind.

4. Durchflussfilter nach Anspruch 3, **dadurch gekennzeichnet, dass**:
- das Bündel und das zweite Bündels jeweils mit ihrem ersten Ende und ihrem zweiten Ende so in dem Filtergehäuse befestigt sind, dass sich ihre Hohlfasern nicht berühren; und
- das Hohlfaserbündel und das zweite Hohlfaserbündel durch einen Verbindungsschlauch außerhalb des Filtergehäuses miteinander verbunden sind.

5. Durchflussfilter nach Anspruch 4, **dadurch gekennzeichnet, dass**:
- das Filtergehäuse eine erste Kammer, in der das Bündel angeordnet ist, und eine gegenüber der ersten Kammer dichte zweite Kammer, in der das zweite Bündel angeordnet ist, umfasst; und
- die Plasma-Ableitung mit der ersten Kammer und eine zweite Zell-Ableitung mit der zweiten Kammer verbunden ist.

6. Durchflussfilter nach einem der Ansprüche 1 bis 5, **dadurch gekennzeichnet, dass** sich das Hohlfaserbündel und das zweite Hohlfaserbündel jeweils vertikal in dem Filtergehäuse erstrecken.

7. Durchflussfilter nach einem der Ansprüche 4 bis 6, **dadurch gekennzeichnet, dass** in dem Verbindungsschlauch ein Sperrventil angeordnet ist.

8. Durchflussfilter nach einem der Ansprüche 1 bis 7, **dadurch gekennzeichnet, dass** die Größe der Poren der ersten feinporigen Membran und die Größe der Poren der zweiten feinporigen Membran verschieden sind.

9. Durchflussfilter nach einem der Ansprüche 1 bis 8, **dadurch gekennzeichnet, dass** die Blutleitung mit einem in einem vorbestimmten Abstand oberhalb des Filtergehäuses angeordneten Blutbeutel verbunden ist.

10. Filteranordnung mit:
a) einem Blutbeutel;
b) einem Leukozytenfilter;
c) einem Durchflussfilter nach einem der Ansprüche 1 bis 9 und einem Zellbeutel; und
d) einem Plasmabeutel
wobei die Elemente a) - d) in dieser Reihenfolge auf zunehmend niedrigeren Niveaus angeordnet sind.

11. Filteranordnung nach Anspruch 10, **dadurch gekennzeichnet, dass** sie eine Byplassleitung zur Umgehung des Leukozytenfilters umfasst.

## Claims

1. Flow filter for separating blood into plasma and cellular constituents, having:
- a filter housing which comprises a blood delivery line, a plasma removal line and a cell removal line,
- a fine-pore membrane which
- comprises a bundle of hollow fibres connected in parallel, which bundle is arranged in the filter housing and has an inflow end connected to the blood delivery line, and
- divides the filter housing into a first flow chamber, which is connected to the cell removal line, and a second flow chamber, which is connected to the plasma removal line, and
- a pressure-adjusting device for adjusting the pressure in the hollow fibres, which pressure-adjusting device comprises a second fine-pore membrane which comprises a second bundle of second hollow fibres which is arranged in the filter housing and has a first end connected to an outflow end of the bundle and a second end connected to the cell removal line.

2. Flow filter according to claim 1, **characterised in that** the pressure-adjusting device comprises a hose line to which an external pressure can be applied.

3. Flow filter according to claim 1 or 2, **characterised in that** the number of hollow fibres and the number of second hollow fibres are different.

4. Flow filter according to claim 3, **characterised in that**:
- the bundle and the second bundle are each fixed with their first end and their second end in the filter housing in such a manner that their hollow fibres do not touch one another; and
- the hollow fibre bundle and the second hollow fibre bundle are connected together outside the filter housing by a connecting hose.

5. Flow filter according to claim 4, **characterised in that**:
- the filter housing comprises a first chamber in which the bundle is arranged, and a second chamber which is tight with respect to the first chamber and in which the second bundle is arranged; and
- the plasma removal line is connected to the first chamber and a second cell removal line is connected to the second chamber.

6. Flow filter according to any one of claims 1 to 5, **characterised in that** the hollow fibre bundle and the second hollow fibre bundle each extend vertically in the filter housing.

7. Flow filter according to any one of claims 4 to 6, **characterised in that** a non-return valve is arranged in the connecting hose.

8. Flow filter according to any one of claims 1 to 7, **characterised in that** the size of the pores of the first fine-pore membrane and the size of the pores of the second fine-pore membrane are different.

9. Flow filter according to any one of claims 1 to 8, **characterised in that** the blood line is connected to a blood bag which is arranged at a predetermined distance above the filter housing.

10. Filter arrangement having:
a) a blood bag;
b) a leukocyte filter;
c) a flow filter according to any one of claims 1 to 9 and a cell bag; and
d) a plasma bag,
wherein elements a) to d) are arranged in that order at increasingly lower levels.

11. Filter arrangement according to claim 10, **characterised in that** it comprises a bypass line for bypassing the leukocyte filter.

## Revendications

1. Filtre d'écoulement pour séparer du sang en plasma des constituants cellulaires, comportant :
- un boîtier de filtre, qui comprend une conduite d'amenée du sang, une conduite d'évacuation du plasma et une conduite d'évacuation des cellules,
- une membrane à pores fins, qui
- comprend un faisceau de fibres creuses montées en parallèle, qui est disposé dans le boîtier de filtre et présente une extrémité d'injection reliée à la conduite d'amenée du sang, et
- subdivise le boîtier du filtre en un premier compartiment d'écoulement, qui est relié à la conduite d'évacuation des cellules, et à un deuxième compartiment d'écoulement, qui est relié à la conduite d'évacuation du plasma, et
- un dispositif de réglage de la pression pour régler la pression dans les fibres creuses, qui comprend une deuxième membrane à pores fins, lequel comprend un deuxième faisceau de deuxièmes fibres creuses, qui est disposé dans le boîtier du filtre, et comprend une première extrémité reliée à l'extrémité d'émission du faisceau et une deuxième extrémité reliée à la conduite d'évacuation des cellules.

2. Filtre d'écoulement selon la revendication 1, **caractérisé en ce que** le dispositif de réglage de la pression comprend un tuyau flexible, sur lequel peut être appliquée une pression externe.

3. Filtre d'écoulement selon la revendication 1 ou 2, **caractérisé en ce que** le nombre des fibres creuses et le nombre des deuxièmes fibres creuses sont différents.

4. Filtre d'écoulement selon la revendication 3, **caractérisé en ce que**
- le faisceau et le deuxième faisceau sont chacun, par leur première extrémité et leur deuxième extrémité, fixés dans le boîtier du filtre de telle sorte que leurs fibres creuses ne se touchent pas ; et
- le faisceau de fibres creuses et le deuxième faisceau de fibres creuses soient reliés l'un à l'autre par un flexible de liaison à l'extérieur du boîtier du filtre.

5. Filtre d'écoulement selon la revendication 4, **caractérisé en ce que**
- le boîtier du filtre comprend une première chambre dans laquelle est disposé le faisceau, et une deuxième chambre, étanche vis-à-vis de la première chambre, dans laquelle est disposé le deuxième faisceau ; et
- la conduite d'évacuation du plasma est reliée à la première chambre, et une deuxième conduite d'évacuation des cellules est reliée à la deuxième chambre.

6. Filtre d'écoulement selon l'une des revendications 1 à 5, **caractérisé en ce que** le faisceau de fibres creuses et le deuxième faisceau de fibres creuses s'étendent chacun verticalement à l'intérieur du boîtier du filtre.

7. Filtre d'écoulement selon l'une des revendications 4 à 6, **caractérisé en ce qu'**un robinet d'arrêt est disposé dans le flexible de liaison.

8. Filtre d'écoulement selon l'une des revendications 1 à 7, **caractérisé en ce que** la grosseur des pores de la première membrane à pores fins et la grosseur des pores de la deuxième membrane à pores fins, sont différentes.

9. Filtre d'écoulement selon l'une des revendications 1 à 8, **caractérisé en ce que** la conduite de sang est reliée à une poche de sang, disposée à une distance prédéfinie au-dessus du boîtier du filtre.

10. Dispositif de filtration, comprenant
a) une poche de sang ;
b) un filtre à leucocytes ;
c) un filtre d'écoulement selon l'une des revendications 1 à 9 et une poche de cellules ; et
d) une poche de plasma,
dans lequel les éléments a) - d) sont disposés dans cet ordre à des niveaux de plus en plus bas.

11. Dispositif de filtration selon la revendication 10, **caractérisé en ce qu'**il comprend une conduite de bipasse pour l'évitement du filtre à leucocytes.
